# NEW EUROPEAN PATENT SPECIFICATION

(11) **EP 1 406 576 B2**
(45) Date of publication and mention of the opposition decision: **29.06.2011**
(45) Mention of the grant of the patent: 03.09.2008
(21) Application number: 02787131.8
(22) Date of filing: 15.07.2002
(51) Int. Cl.: A61K 8/42, A61Q 5/02, A61Q 5/00

(54) **HAIR AND/OR SCALP TREATMENT COMPOSITIONS**
ZUSAMMENSETZUNGEN FÜR DIE BEHANDLUNG VON HAAREN UND/ODER KOPFHAUT
COMPOSITION DE TRAITEMENT DU CUIR CHEVELU ET/OU DES CHEVEUX

(30) Priority: 18.07.2001 EP 01306166
(43) Date of publication of application: 14.04.2004
(60) Divisional application: 07002049.0
(73) Proprietor: Unilever PLC, London EC4Y 0DY (GB); Unilever N.V., 3013 AL Rotterdam (NL)
(72) Inventor: BAILEY, Peter Lawrence, Bebington, Merseyside CH63 3JW (GB); LITTLE, Christopher John, Pharmacy Department, 4062 New Plymouth (NZ); MCGLONE, Francis, Bebington, Wirral, Merseyside CH63 3JW (GB); RUKWIED, Roman, Bebington, Wirral, Merseyside CH63 3JW (GB); WATKINSON, Allan, Billingham, Cleveland TS23 1YN (GB)
(74) Representative: McHugh, Paul Edward
(86) International application number: PCT/EP2002/007904
(87) International publication number: WO 2003/007901

(56) References cited:
- WO-A-00/07627
- WO-A-00/16756
- WO-A-01/04083
- WO-A-01/24645
- WO-A-01/85158
- WO-A-92/10995
- WO-A-97/00860
- WO-A1-00/24367
- WO-A1-01/10434
- WO-A1-97/29733
- DE-T2- 69 326 188
- GB-A- 1 230 413
- GB-A- 2 338 413
- US-A- 2 773 835
- US-A- 5 154 847
- US-A- 5 730 965
- B.ZIOLKOWSKY: 'Emulsionen in Kosmetik und Pharmazie', 1985, H.ZIOLKOWSKY KG, AUSBURG pages 308,360 - 390 & COGNIS PRODUCT DATA SHEET COMPERLAN KD 03 January 2002 - 03 January 2002, pages 1 - 2 & FR 2 685 638 A1 02 July 1993
- 'Römpp Chemie Lexikon', vol. 3, 1995, GEORG THIEME VERLAG, STUTTGART article DR. ELISABETH HILLEN-MASKE, page 2294

## Description

This invention relates to hair and/or scalp treatment compositions and to their use in the cosmetic treatment of hair and/or the treatment or prevention of the symptoms of dandruff. The invention also relates to the use of certain compounds for the treatment of scalp itch.

### Background

It is widely believed that *Malassezia* yeasts, such as *Malassezia furfur,* are the main cause of dandruff. However, it is unclear why some people suffer from this condition while others do not. What is known is that increasing the level of *Malassezia* on the scalp does not automatically lead to dandruff. This suggests that *Malassezia* is necessary but not sufficient to cause the condition.

The main, if not only, intervention strategy used on the market currently for the treatment of dandruff is the topical application of antifungals such as zinc pyrithione (ZnPTO), octopirox and ketoconazole which are normally delivered from a shampoo. These antifungal agents remove (or at least reduce the level of) the *Malassezia* from the scalp, and provide effective treatment of the dandruff condition.

Although clinically proven to be effective in treating the clinical symptoms of dandruff over a two to four week period, there remains a need to treat the main symptoms of dandruff more effectively and rapidly. The main symptoms of dandruff are visible skin flakes in the hair and on the shoulders and scalp itch. Scalp itch is perceived as being a particular problem in certain parts of the world, for example it is the main symptom of dandruff in China, South-East Asia and India.

US 5 154 847 discloses an antidandruff shampoo composition comprising an antidandruff active, suspending agents and detergent surfactants which may include palmitoyl ethanolamide.

As well as treating the clinical signs of dandruff, therefore, there remains a need for providing rapid relief from scalp itch for dandruff sufferers.

Mankind has sought therapeutic means to relieve pain and suffering ever since records have been kept. Early remedies involved the administration of natural products, commonly by ingestion, topical application or inhalation, presumably as a result of empirical observations. For example, *Cannabis sativa,* colloquially called cannabis, or extracts therefrom, has been employed therapeutically for approximately 3000 years, being first mentioned in the time of Emperor Shen Nung in the *Chinese Compendium of Medicine.* It has subsequently been suggested that its perceived efficacy can be due to the action of Δ⁹-tetrahydrocannabinol. Cannabinoid agents have been asserted to have anti-inflammatory actions (A.W. Wirth et al., Life Sci. 26, 1991-1995 (1980)). More recent research has shown that the biological actions of cannabis are mediated by two similar, but distinct, types of membrane receptor which have been termed Cannabinoid Receptor 1 (CB1R) and Cannabinoid Receptor 2 (CB2R) (Matsuda et al, Nature 346, 561-564 (1990), Munro et al, Nature, 365, 61-65 (1993)).

CB1R is by far the predominant form in the central nervous system, whereas both CB1R and CB2R are located in the peripheral tissues, including the skin. Some investigations have been made to locate natural endogenous ligands that can activate one or other of these receptors. Herein a reference to a CB receptor (CBR) activator includes either or both CB1R and CB2R activators.

Arachidonylethanolamide (anandamide) has been stated to exhibit a greater affinity for the CB1R compared with CB2R and is believed to be the endogenous CB1R agonist (Pertwee et al, Br. J. Pharmacol. 105, 980-984 (1992)). It has been suggested that palmitoylethanolamide and 2-arachidonyl-glycerol are putative CB2R agonists (Facci et al, Proc. Natl. Acad. Sci. 92, 3376-3380 (1995), Sugiura et al., J. Biol. Chem. 275, 605-612 (2000)). Ligands that activate both CB1R and CB2R have been shown to be analgesic, whether applied systemically or cutaneously (Calignano et al, Nature,394, 277-280 (1998)).

It is known that palmitoyl monoethanolamide can be used as a pearlising agent in shampoos. PL-A-93518 is an example of such a disclosure.

Therapeutic uses have already been contemplated involving cannabinoid receptors (CB receptors or CBRs). For example, US-A-5990170 (Della Valle et al) teaches the therapeutic use of a range of selected mono and dicarboxylic acid amides which bind the CB2R in order to treat diseases connected with this receptor. EP-A-550006 (Della Valle et al) teaches a range of N-acyl derivatives of amino alcohols to be used for the treatment of pathologies characterised by the degranulation of mast cells. US-A-5679667 describes the use of amino alcohols-N-acyl derivatives as therapeutic agents against neurogenic endoneural edema of the peripheral nerve. However, none of these references disclose or contemplate in any way hair and/or scalp treatment formulations or the use of any of the actives disclosed therein in such formulations.

WO 92/10995 discloses a hair lotion for the treatment of dandruff that contains glyceryl monolinoleate. The glyceryl monolinoleate is described as having useful activity in the treatment of a number of different skin conditions, including dandruff. There is no mention of shampoo compositions or of the activity of the compound as a CB (cannabinoid) receptor activating agent.

WO 00/16756 relates to the use of N-acylvanillinamide derivatives capable of activating the peripheral receptor CB1 of cannabinoids. Examples given in the document include a dermatological cream and a hair lotion. There is no mention in the document of the treatment of dandruff or of a shampoo or conditioner composition.

The present invention aims to provide improved compositions for the treatment or prevention of dandruff and/or the symptoms of dandruff.

### Summary of the invention

According to the invention there is provided a hair and/or scalp treatment composition, comprising an antidandruff agent selected from zinc pyrithione, climbazole, ketoconazole, octopirox and mixtures thereof, a CB receptor activating agent being palmitoylethanolamide and a cosmetically acceptable diluent or carrier.

### Detailed Description of the Invention and Preferred Embodiments

By the employment of palmitoylethanolamide as CBR activating agent in an effective amount in a hair and/or scalp treatment formulation, it is possible for users of such formulations to experience rapid relief from scalp itch. It has been found that a rapid relief from scalp itch leads to an overall improvement in the user's perception of their dandruff condition.

The capability of a compound to act as a CBR activating agent can be determined using a topical application test on human skin in which the irritant effect of a predetermined concentration of a known irritant, for example a known pruritic agent, such as histamine, is counteracted by chosen amounts of the target compound. Alternatively or additionally, the effectiveness of a material to act as a CBR activating agent can be determined by incorporating it in a hair and/or scalp treatment composition and observing the extent to which scalp itch is diminished.

It can be convenient to employ a topical application test as a way of demonstrating whether the test material would be effective as a CBR activating agent. In one such test employing the control conditions described subsequently herein, human skin is contacted with a control or test material under a water-impermeable patch for a specified length of time, such as 24 hours and then challenged with a predetermined dose of a known irritant such as histamine applied by iontophoresis at a specified current for a specified period, e.g. 10 seconds at 50µA, which is counteracted by application of a test material within a range of concentrations so as to be able to identify that at which the material becomes effective. By plotting the perceived strength of the response against time, a test value can be obtained which comprises the area under the curve. This area is normally that from time 0 to 5 minutes and the base line at 0 to the response curve. In an alternative, but related, test procedure, a cream formulation is applied topically to the skin, for example for a period of 4 hours rather than being covered by a patch, and is then challenged in the same way.

The measured activity of test material can vary between subjects. Accordingly, an averaged result should be employed. The test material can be considered to pass the histamine test at its chosen concentration when the averaged test value from histamine plus the test material is less than 80% of the control test value, i.e. that obtained from histamine without the test material under the same test conditions. Preferably, the material and its amount are chosen in combination such that the resultant averaged test value is not higher than 50% of the control test value.

In such a test, the difference necessary for it to be statistically significant decreases as the number of subjects in the test increases. For the most effective CBR activating agents, a small number of subjects can demonstrate a positive result, whereas for less active agents, it is desirable to employ a larger number such as at least 20 subjects and preferably about 50.

Herein a material is considered to be a CBR activating agent, if it passes the foregoing test or any other test method for CBRs described herein.

It is particularly desirable to identify and employ a compound that can act as an activating agent for a CBR ie for either or both CB2R and CB1R, on account of the presence of both CB1R and CB2R sites in skin.

Examples of CBR activating agents include:-
2-arachidonyl-glycerol
1-arachidonyl-glycerol
3-arachidonyl-glycerol
2-linoleoyl-glycerol
2-linolenoyl-glycerol
2-eicosatrienoyl-glycerol
2-eicosatetraenoyl-glycerol
2-eicosapentenoyl-glycerol
2-eicosahexaenoyl-glycerol
Palmitoylethanolamide
(6aR)-trans-3-(1,1-Dimethylheptyl)-6a,7,10,10a-tetrahydro-1-hydroxy-6,6-dimethyl-6H-dibenzo[b,d]pyran-9-methanol,
sometimes called HU-210 herein;
Indomethacin morpholinylamide sometimes abbreviated to IMMA herein;
mesylate: (R)-(+)-[2,3-Dihydro-5-methyl-3-(4-morpholinylmethyl)pyrrolo[1,2,3-de]-1,4-benzoxazin-6-yl]-1-naphthalenylmethanone, sometimes called WIN55212-2 herein; (-)-cis-3-[2-Hydroxy-4-(1,1-dimethylheptyl)phenyl]-trans-4-(3-hydroxypropyl) cyclohexanol, sometimes called CP55940 herein;
R-(+)-Methanandamide: [R-(all-Z)]-N-(2-Hydroxy-1-methylethyl)-5,8,11,14-eicosatetraenamide;
Arachidonyl-2'-chloroethylamide: (all Z)-N-(2-cycloethyl)-5,8,11,14-eicosatetraenamide): and
Arachidonylcyclopropylamide: (all Z)-N-(cyclopropyl)-5,8,11,14-eicosatetraenamide.

Mixtures of two or more of said CBR activating agents can be employed herein, such as a combination of palmitoylethanolamide with an aforementioned glycerol derivative, for example in a weight ratio of from 10:1 to 1:10.

The amount of CBR activating agent in the compositions of the invention is selected in the range of from at least 0.51%, and often to not more than 20%, more preferably in some embodiments from 0.1% and in those or other embodiments up to 10%, as herein being by weight based on the formulation or base formulation, as the case may be, unless otherwise stated. In a number of practical formulations, the concentration of CBR activating agent is not more than 5%, and particularly from 0.25 to 2 wt%.

The compositions according to the invention comprise an antidandruff agent. The antidandruff agent is a different compound from the CBR activating agent. Antidandruff agents are compounds that are active against dandruff and are typically antimicrobial agents, preferably antifungal agents. Preferably, compositions according to the invention comprise from 0.01% to 30% by weight, more preferably 0.1% to 10%, most preferably 0.5 to 2% by weight of the antidandruff agent.

Suitable antidandruff agents include compounds selected from zinc pyrithione, climbazole, ketoconazole, octopirox and mixtures thereof.

The preferred antifungal agent is zinc pyrithione (ZnPTO) which, on account of its relative insolubility in aqueous systems, is generally used in hair treatment compositions as a particulate dispersion. The zinc pyrithione may be used in any particle form including, for example, crystalline forms such as platelets and needles and amorphous, regularly or irregularly shaped particles. If zinc pyrithione is present in the composition, a suspending agent is preferably used to prevent or inhibit the settling of the particles out of the composition. The average particle diameter of the zinc pyrithione particles (ie, their maximum dimension) is typically from about 0.2 to about 50 µm, preferably from about 0.4 to about 10 µm, more preferably from 0.4 to 1µm.

Antifungal agents typically display a minimum inhibitory concentration of about 50 mg/ml or less against *Malassezia.*

If the antifungal agent is soluble in aqueous systems, it may be present in solution in a composition used in the invention.

The compositions of the invention may contain a cosmetically acceptable diluent or carrier. Suitable diluents and/or carriers are well-known in the art and include, for example, water and water-miscible organic solvents (such as ethanol, ethylene glycol and propylene glycol). Other diluents and/or carriers are described hereinafter in the context of particular exemplary product forms.

### Product Forms

Compositions of the present invention are typically for topical application to the scalp (including by simultaneous application to the hair) and may be formulated as transparent or opaque emulsions, lotions, creams, pastes or gels. Particularly preferred product forms are shampoos and conditioners, especially shampoos.

### Shampoo Compositions

A particularly preferred hair treatment composition in accordance with the invention is a shampoo composition.

Such a shampoo composition will comprise one or more cleansing surfactants which are cosmetically acceptable and suitable for topical application to the hair. Further surfactants may be present as an additional ingredient if sufficient for cleansing purposes is not provided as emulsifier for any emulsified components in the composition, e.g. emulsified silicones. It is preferred that shampoo compositions of the invention comprise at least one further surfactant (in addition to that used as emulsifying agent) to provide a cleansing benefit.

Suitable cleansing surfactants, which may be used singularly or in combination, are selected from anionic, amphoteric and zwitterionic surfactants, and mixtures thereof. The cleansing surfactant may be the same surfactant as the emulsifier, or may be different.

Examples of anionic surfactants are the alkyl sulphates, alkyl ether sulphates, alkaryl sulphonates, alkanoyl isethionates, alkyl succinates, alkyl sulphosuccinates, N-alkyl sarcosinates, alkyl phosphates, alkyl ether phosphates, alkyl ether carboxylates, and alpha-olefin sulphonates, especially their sodium, magnesium, ammonium and mono-, di- and triethanolamine salts. The alkyl and acyl groups generally contain from 8 to 18 carbon atoms and may be unsaturated. The alkyl ether sulphates, alkyl ether phosphates and alkyl ether carboxylates may contain from 1 to 10 ethylene oxide or propylene oxide units per molecule.

Typical anionic surfactants for use in shampoos of the invention include sodium oleyl succinate, ammonium lauryl sulphosuccinate, ammonium lauryl sulphate, sodium dodecylbenzene sulphonate, triethanolamine dodecylbenzene sulphonate, sodium cocoyl isethionate, sodium lauryl isethionate and sodium N-lauryl sarcosinate. The most preferred anionic surfactants are sodium lauryl sulphate, triethanolamine monolauryl phosphate, sodium lauryl ether sulphate 1 EO, 2EO and 3EO, ammonium lauryl sulphate and ammonium lauryl ether sulphate 1EO, 2EO and 3EO.

Examples of amphoteric and zwitterionic surfactants include alkyl amine oxides, alkyl betaines, alkyl amidopropyl betaines, alkyl sulphobetaines (sultaines), alkyl glycinates, alkyl carboxyglycinates,alkyl amphopropionates, alkylamphoglycinates, alkyl amidopropyl hydroxysultaines, acyl taurates and acyl glutamates, wherein the alkyl and acyl groups have from 8 to 19 carbon atoms. Typical amphoteric and zwitterionic surfactants for use in shampoos of the invention include lauryl amine oxide, cocodimethyl sulphopropyl betaine and preferably lauryl betaine, cocamidopropyl betaine and sodium cocamphopropionate.

The shampoo composition can also include co-surfactants, to help impart aesthetic, physical or cleansing properties to the composition. A preferred example is a nonionic surfactant, which can be included in an amount ranging from 0% to about 5% by weight of the total composition.

For example, representative nonionic surfactants that can be included in shampoo compositions of the invention include condensation products of aliphatic (C₈ - C₁₈) primary or secondary linear or branched chain alcohols or phenols with alkylene oxides, usually ethylene oxide and generally having from 6 to 30 ethylene oxide groups.

Other representative nonionics include mono- or di-alkyl alkanolamides. Examples include coco mono- or diethanolamide and coco mono-isopropanolamide.

Further nonionic surfactants which can be included in shampoo compositions of the invention are the alkyl polyglycosides (APGs). Typically, the APG is one which comprises an alkyl group connected (optionally via a bridging group) to a block of one or more glycosyl groups. Preferred APGs are defined by the following formula:

RO - (G)ₙ

wherein R is a branched or straight chain alkyl group which may be saturated or unsaturated and G is a saccharide group.

R may represent a mean alkyl chain length of from about C₅ to about C₂₀. Preferably R represents a mean alkyl chain length of from about C₈ to about C₁₂. Most preferably the value of R lies between about 9.5 and about 10.5. G may be selected from C₅ or C₆ monosaccharide residues, and is preferably a glucoside. G may be selected from the group comprising glucose, xylose, lactose, fructose, mannose and derivatives thereof. Preferably G is glucose.

The degree of polymerisation, n, may have a value of from about 1 to about 10 or more. Preferably, the value of n lies in the range of from about 1.1 to about 2. Most preferably the value of n lies in the range of from about 1.3 to about 1.5.

Suitable alkyl polyglycosides for use in the invention are commercially available and include for example those materials identified as: Oramix N_{S}10 ex Seppic; Plantaren 1200 and Plantaren 2000 ex Henkel.

The total amount of surfactant (including any co-surfactant, and/or any emulsifier) in shampoo compositions of the invention is generally from 0.1 to 50% by weight (such as 3% to 50% by weight), preferably from 5 to 30%, more preferably from 10% to 25% by weight of the total shampoo composition.

A cationic deposition polymer is a preferred ingredient in shampoo compositions of the invention, for enhancing conditioning performance of the shampoo. By "deposition polymer" is meant an agent which enhances deposition of the silicone component from the shampoo composition onto the intended site during use, i.e. the hair and/or the scalp.

The deposition polymer may be a homopolymer or be formed from two or more types of monomers. The molecular weight of the polymer (in g/mol) will generally be between 5 000 and 10 000 000, typically at least 10 000 and preferably in the range 100 000 to about 2 000 000. The polymers will have cationic nitrogen containing groups such as quaternary ammonium or protonated amino groups, or a mixture thereof.

The cationic nitrogen-containing group will generally be present as a substituent on a fraction of the total monomer units of the deposition polymer. Thus when the polymer is not a homopolymer it can contain spacer non-cationic monomer units. Such polymers are described in the CTFA Cosmetic Ingredient Directory, 3rd'edition. The ratio of the cationic to non-cationic monomer units is selected to give a polymer having a cationic charge density in the required range.

Suitable cationic deposition polymers include, for example, copolymers of vinyl monomers having cationic amine or quaternary ammonium functionalities with water soluble spacer monomers such as (meth)acrylamide, alkyl and dialkyl (meth)acrylamides, alkyl (meth)acrylate, vinyl caprolactone and vinyl pyrrolidine. The alkyl and dialkyl substituted monomers preferably have C1-C7 alkyl groups, more preferably C1-3 alkyl groups. Other suitable spacers include vinyl esters, vinyl alcohol, maleic anhydride, propylene glycol and ethylene glycol.

The cationic amines can be primary, secondary or tertiary amines, depending upon the particular species and the pH of the composition. In general secondary and tertiary amines, especially tertiary, are preferred.

Amine substituted vinyl monomers and amines can be polymerized in the amine form and then converted to ammonium by quaternization.

The cationic deposition polymers can comprise mixtures of monomer units derived from amine- and/or quaternary ammonium-substituted monomer and/or compatible spacer monomers.

Suitable cationic deposition polymers include, for example:
- copolymers of 1-vinyl-2-pyrrolidine and 1-vinyl-3-methylimidazolium salt (e.g. chloride salt), referred to in the industry by the Cosmetic, Toiletry, and Fragrance Association, (CTFA) as Polyquaternium-16. This material is commercially available from BASF Wyandotte Corp. (Parsippany, NJ, USA) under the LUVIQUAT tradename (e.g. LWIQUAT FC 370);
- copolymers of 1-vinyl-2-pyrrolidine and dimethylaminoethyl methacrylate, referred to in the industry (CTFA) as Polyquaternium-11. This material is available commercially from Gaf Corporation (Wayne, NJ, USA) under the GAFQUAT tradename (e.g., GAFQUAT 755N);
- cationic diallyl quaternary ammonium-containing polymers including, for example, dimethyldiallylammonium chloride homopolymer and copolymers of acrylamide and dimethyldiallylammonium chloride, referred to in the industry (CTFA) as Polyquaternium 6 and Polyquaternium 7, respectively;
- mineral acid salts of amino-alkyl esters of homo-and copolymers of unsaturated carboxylic acids having from 3 to 5 carbon atoms, (as described in U.S. Patent 4,009,256);
- cationic polyacrylamides(as described in WO95/22311).

Other cationic deposition polymers that can be used include cationic polysaccharide polymers, such as cationic cellulose derivatives, cationic starch derivatives, and cationic guar gum derivatives.

Cationic polysaccharide polymers suitable for use in compositions of the invention include those of the formula:

A-O- [R-N⁺(R¹) (R²) (R³) X⁻],

wherein: A is an anhydroglucose residual group, such as a starch or cellulose anhydroglucose residual. R is an alkylene, oxyalkylene, polyoxyalkylene, or hydroxyalkylene group, or combination thereof. R¹, R² and R³ independently represent alkyl, aryl, alkylaryl, arylalkyl, alkoxyalkyl, or alkoxyaryl groups, each group containing up to about 18 carbon atoms. The total number of carbon atoms for each cationic moiety (i.e., the sum of carbon atoms in R¹, R² and R³) is preferably about 20 or less, and X is an anionic counterion.

Cationic cellulose is available from Amerchol Corp. (Edison, NJ, USA) in their Polymer JR (trade mark) and LR (trade mark) series of polymers, as salts of hydroxyethyl cellulose reacted with trimethyl ammonium substituted epoxide, referred to in the industry (CTFA) as Polyquaternium 10. Another type of cationic cellulose includes the polymeric quaternary ammonium salts of hydroxyethyl cellulose reacted with lauryl dimethyl ammonium-substituted epoxide, referred to in the industry (CTFA) as Polyquaternium 24. These materials are available from Amerchol Corp. (Edison, NJ, USA) under the tradename Polymer LM-200.

Other suitable cationic polysaccharide polymers include quaternary nitrogen-containing cellulose ethers (e.g. as described in U.S. Patent 3,962,418), and copolymers of etherified cellulose and starch (e.g. as described in U.S. Patent 3,958,581).

A particularly suitable type of cationic polysaccharide polymer that can be used is a cationic guar gum derivative, such as guar hydroxypropyltrimonium chloride (Commercially available from Rhodia (formerly Rhone-Poulenc) in their JAGUAR trademark series).

Examples are JAGUAR C13S, which has a low degree of substitution of the cationic groups and high viscosity. JAGUAR C15, having a moderate degree of substitution and a low viscosity, JAGUAR C17 (high degree of substitution, high viscosity), JAGUAR C16, which is a hydroxypropylated cationic guar derivative containing a low level of substituent groups as well as cationic quaternary ammonium groups, and JAGUAR 162 which is a high transparency, medium viscosity guar having a low degree of substitution.

Preferably the cationic deposition polymer is selected from cationic cellulose and cationic guar derivatives. Particularly preferred deposition polymers are JAGUAR C13S, JAGUAR C15, JAGUAR C17 and JAGUAR C16 and JAGUAR C162.

The cationic deposition polymer will generally be present at levels of from 0.001 to 5%, preferably from about 0.01 to 1%, more preferably from about 0.02% to about 0.5% by weight of the total composition.

### Solid Active Agents

Solid active agents include pigment particles, such as solid dyes or colorants suitable for application to hair, and metal colloids.

### Aesthetic Agents

Hair treatment compositions such as shampoos and conditioners are frequently opacified or pearlised to enhance consumer appeal.

Examples of opacifying agents include higher fatty alcohols (e.g. cetyl, stearyl, arachidyl and behenyl), solid esters (e.g. cetyl palmitate, glyceryl laurate, stearamide MEA-stearate), high molecular weight fatty amides and alkanolamides and various fatty acid derivatives such as propylene glycol and polyethylene glycol esters. Inorganic materials used to opacify hair treatment compositions include magnesium aluminium silicate, zinc oxide, and titanium dioxide.

Pearlescing agents typically form thin, platelet-type crystals in the composition, which act like tiny mirrors. This gives the pearl lustre effect. Some of the opacifying agents listed above may also crystallise as pearlescing agents, depending on the media in which they are used and the conditions employed.

Typical pearlescing agents may be selected from C16-C22 fatty acids (e.g. stearic acid, myristic acid, oleic acid and behenic acid), esters of C16-C22 fatty acid with alcohols and esters of C16-C22 fatty acid incorporating such elements as alkylene glycol units. Suitable alkylene glycol units may include ethylene glycol and propylene glycol. However, higher alkylene chain length glycols may be employed. Suitable higher alkylene chain length glycols include polyethylene glycol and polypropylene glycol.

Examples are polyethylene glycol mono or diesters of C16-C22 fatty acids having from 1 to 7 ethylene oxide units, and ethylene glycol esters of C16-C22 fatty acids. Preferred esters include polyethylene glycol distearates and ethylene glycol distearates. Examples of a polyethylene glycol distearate available commercially are EUPERLAN PK900 (ex Henkel) or GENAPOL TS (ex Hoechst). An example of an ethylene glycol distearate is EUPERLAN PK3000 (ex Henkel).

Other pearlescing agents include alkanolamides of fatty acids having from 16 to 22 carbon atoms, (e.g. stearic monoethanolamide, stearic diethanolamide, stearic monoisopropanolamide and stearic monoethanolamide stearate); long chain esters of long chain fatty acids (e.g. stearyl stearate, cetyl palmitate); glyceryl esters (e.g. glyceryl distearate),long chain esters of long chain alkanolamides (e.g. stearamide DEA distearate, stearamide MEA stearate), and alkyl (C18-C22) dimethyl amine oxides (e.g. stearyl dimethyl amine oxide) .

Further suitable pearlescing agents include inorganic materials such as nacreous pigments based on the natural mineral mica. An example is titanium dioxide coated mica. Particles of this material may vary in size from 2 to 150 microns in diameter. In general, smaller particles give rise to a pearly appearance, whereas particles having a larger average diameter will result in a glittery composition.

Suitable titanium dioxide coated mica particles are those sold under the trade names TIMIRON (Merck) or FLAMENCO (Mearl).

The level of opacifying or pearlescing agent employed in compositions of the invention is generally from 0.01 to 20%, preferably 0.01 to 5%, more preferably from 0.02 to 2% by weight of the total composition.

Gas (e.g. air) bubbles represent another type of suspended phase that may be introduced into a hair treatment composition for aesthetic purposes. When evenly sized and homogeneously dispersed in the composition, these can enhance consumer appeal - a typical application is in a transparent or translucent composition such as a hair styling gel.

### Conditioners

Compositions in accordance with the invention may also be formulated as conditioners for the treatment of hair (typically after shampooing) and subsequent rinsing.

Such a conditioner will comprise one or more conditioning surfactants which are cosmetically acceptable and suitable for topical application to the hair. Suitable conditioning surfactants are selected from cationic surfactants, used singly or in admixture. Examples include quaternary ammonium hydroxides or salts thereof, e.g. chlorides.

Suitable cationic surfactants for use in hair conditioners of the invention include cetyltrimethylammonium chloride, behenyltrimethylammonium chloride, cetylpyridinium chloride, tetramethylammonium chloride, tetraethylammonium chloride, octyltrimethylammonium chloride, dodecyltrimethylammonium chloride, hexadecyltrimethylammonium chloride, octyldimethylbenzylammonium chloride, decyldimethylbenzylammonium chloride, stearyldimethylbenzylammonium chloride, didodecyldimethylammonium chloride, dioctadecyldimethylammonium chloride, tallowtrimethylammonium chloride, cocotrimethylammonium chloride, and the corresponding hydroxides thereof. Further suitable cationic surfactants include those materials having the CTFA designations Quaternium-5, Quaternium-31 and Quaternium-18. Mixtures of any of the foregoing materials may also be suitable. A particularly useful cationic surfactant for use in hair conditioners of the invention is cetyltrimethylammonium chloride, available commercially, for example as GENAMIN CTAC, ex Hoechst Celanese.

In conditioners of the invention, the level of cationic surfactant is preferably from 0.01 to 10%, more preferably 0.05 to 5%, most preferably 0.1 to 2% by weight of the composition.

Conditioners of the invention advantageously incorporate a fatty alcohol. The combined use of fatty alcohols and cationic surfactants in conditioning compositions is believed to be especially advantageous, because this leads to the formation of a lamellar phase, in which the cationic surfactant is dispersed.

Representative fatty alcohols comprise from 8 to 22 carbon atoms, more preferably 16 to 20. Examples of suitable fatty alcohols include cetyl alcohol, stearyl alcohol and mixtures thereof. The use of these materials is also advantageous in that they contribute to the overall conditioning properties of compositions of the invention.

The level of fatty alcohol in conditioners of the invention is conveniently from 0.01 to 10%, preferably from 0.1 to 5% by weight of the composition. The weight ratio of cationic surfactant to fatty alcohol is suitably from 10:1 to 1:10, preferably from 4:1 to 1:8, optimally from 1:1 to 1:4.

### Conditioning Agents

The compositions which may be used in the invention may contain a conditioning agent. As used herein, the term "conditioning agent" includes any material which is used to give a particular conditioning benefit to hair and/or skin. For example, in compositions for use in washing hair, such as shampoos and conditioners, suitable materials are those which deliver one or more benefits relating to shine, softness, combability, wet-handling, anti-static properties, protection against damage, body, volume, stylability and manageability.

Preferred conditioning agents for use in the present invention include emulsified silicones, used to impart for example wet and dry conditioning benefits to hair such as softness, smooth feel and ease of combability.

Various methods of making emulsions of particles of silicones for use in the invention are available and are well known and documented in the art.

The viscosity of the silicone itself (not the emulsion or the final washing composition) preferably ranges from 10,000 cps to 5 million cps. The viscosity can be measured by means of a glass capillary viscometer as set out further in Dow Corning Corporate Test Method CTM004 July 20 1970.

Suitable silicones include polydiorganosiloxanes, in particular polydimethylsiloxanes which have the CTFA designation dimethicone. An example is dimethicone fluid having a viscosity of up to 100,000 centistokes at 25° C, which is available commercially from the General Electric Company as the Viscasil series and from Dow Corning as the DC 200 series.

Aminofunctional silicones which have the CTFA designation amodimethicone, are also suitable for use in the compositions of the invention, as are polydimethyl siloxanes having hydroxyl end groups, which have the CTFA designation dimethiconol.

Also suitable are silicone gums. "Silicone gum" denotes polydiorganosiloxanes having a molecular weight of from 200,000 to 1,000,000 and specific examples include dimethicone gums, dimethiconol gums, polydimethyl siloxane/diphenyl/methylvinylsiloxane copolymers, polydimethylsiloxane/methylvinylsiloxane copolymers and mixtures thereof. Examples include those materials described in US Pat. No. 4,152,416 (Spitzer), and on General Electric Silicone Rubber product Data Sheet SE 30, SE 33, SE 54 and SE 76.

Also suitable for use in the present invention are silicone gums having a slight degree of cross-linking, as are described for example in WO 96/31188. These materials can impart body, volume and stylability to hair, as well as good wet and dry conditioning.

Preferred emulsified silicones for use in compositions of the invention have an average silicone particle size in the composition of less than 100, preferably less than 30, more preferably less than 20 microns, most preferably less than 10 microns.

Particle size may be measured by means of a laser light scattering technique, using a 2600D Particle Sizer from Malvern Instruments.

Suitable silicone emulsions for use in the invention are commercially available in a pre-emulsified form. This is particularly preferred since the pre-formed emulsion can be incorporated into the washing composition by simple mixing.

Examples of suitable pre-formed emulsions include emulsions DC2-1766 and DC2-1784, available from Dow Corning. These are emulsions of dimethiconol. Cross-linked silicone gums are also available in a pre-emulsified form, which is advantageous for ease of formulation. A preferred example is the material available from Dow Corning as DC X2-1787, which is an emulsion of cross-linked dimethiconol gum.

The amount of silicone incorporated into the compositions of the invention depends on the level of conditioning desired and the material used. A preferred amount is from 0.01 to about 10% by weight of the total composition although these limits are not absolute. The lower limit is determined by the minimum level to achieve conditioning and the upper limit by the maximum level to avoid making the hair and/or skin unacceptably greasy. We have found that an amount of silicone of from 0.5 to 1.5% by weight of the total composition, is a particularly suitable level.

A further preferred class of conditioning agents are peralk(en)yl hydrocarbon materials, used to enhance the body, volume and stylability of hair.

EP 567 326 and EP 498 119 describe suitable peralk(en)yl hydrocarbon materials for imparting stylability and enhanced body to hair. Preferred materials are polyisobutylene materials available from Presperse, Inc. under the PERMETHYL trade name.

The amount of per-alk(en)yl hydrocarbon material incorporated into the compositions of the invention depends on the level of body and volume enhancement desired and the specific material used. A preferred amount is from 0.01 to about 10% by weight of the total composition although these limits are not absolute. The lower limit is determined by the minimum level to achieve the body and volume enhancing effect and the upper limit by the maximum level to avoid making the hair unacceptably stiff. We have found that an amount of per-alk(en)yl hydrocarbon material of from 0.5 to 2% by weight of the total composition is a particularly suitable level.

### Optional Ingredients

Compositions of this invention may contain any other ingredient normally used in hair treatment formulations. These other ingredients may include viscosity modifiers, preservatives, colouring agents, polyols such as glycerine and polypropylene glycol, chelating agents such as EDTA, antioxidants, fragrances, and sunscreens. Each of these ingredients will be present in an amount effective to accomplish its purpose. Generally these optional ingredients are included individually at a level of up to about 5% by weight of the total composition.

Preferably, compositions of this invention also contain adjuvants suitable for hair care. Generally such ingredients are included individually at a level of up to 2%, preferably up to 1%, by weight of the total composition.

Among suitable hair care adjuvants, are:
(i) natural hair root nutrients, such as amino acids and sugars. Examples of suitable amino acids include arginine, cysteine, glutamine, glutamic acid, isoleucine, leucine, methionine, serine and valine, and/or precursors and derivatives thereof. The amino acids may be added singly, in mixtures, or in the form of peptides, e.g. di- and tripeptides. The amino acids may also be added in the form of a protein hydrolysate, such as a keratin or collagen hydrolysate. Suitable sugars are glucose, dextrose and fructose. These may be added singly or in the form of, e.g. fruit extracts. A particularly preferred combination of natural hair root nutrients for inclusion in compositions of the invention is isoleucine and glucose. A particularly preferred amino acid nutrient is arginine.
(ii) hair fibre benefit agents. Examples are:
   - ceramides, for moisturising the fibre and maintaining cuticle integrity. Ceramides are available by extraction from natural sources, or as synthetic ceramides and pseudoceramides. A preferred ceramide is Ceramide II, ex Quest. Mixtures of ceramides may also be suitable, such as Ceramides LS, ex Laboratoires Serobiologiques

The invention will now be further illustrated by the following, non-limiting examples. In the examples and throughout the specification, all percentages are percentages by weight unless indicated otherwise.

### EXAMPLES

### Example 1 (for information only)

### Effect of topically applied HU-210 on histamine-induced itch.

In this demonstration, a material was tested to see if it was a CBR activating agent. HU210 was dissolved in Aqueous Cream BP at a concentration of 0.5% and 0.05% (w/v) and topically applied to the volar forearm skin: five applications of 50µl per 3 cm² area and itching perception was determined after 4 hours. The control site contained the vehicle alone and the study was performed in a double-blind fashion. The cream covered area was then treated with histamine applied by iontophoresis to induce itch. In summary, a 1% histamine solution in 1% methylcellulose was placed in the iontophoretic chamber and a 10 sec burst of 50µA was applied using an Iontophoresis Controller MIC-e (Moors Instruments, of Axminster, U.K.). After application of the current, each subject indicated the intensity of the itch sensation, by moving a slider to the appropriate point on a Visual Analogue Scale (VAS), which ranges from threshold sensation (0) to maximal itch (10), to indicate the subject's itch response. VAS readings were taken for 5 minutes following histamine application and plotted against the time of the reading. The VAS value shown in subsequent tables of results comprises the area under the curve from 0 to 5 minutes. The results are summarised in Table 1 below. The formulation of the Aqueous Cream B.P. comprised
cetylstearyl alcohol 8.1%w/w
sodium lauryl sulphate 0.9% w/w
liquid paraffin 6.0%
white soft paraffin 15.0% w/w
water - balance.

**Table 1**

| Treatment | | | Itch score (area under the curve) | Itch score % of control |
|---|---|---|---|---|
| | Vehicle | HU-210 amount | | |
| Control | yes | 0 | 1634 | 100 |
| 1.1 | yes | 0.05% | 101 | 7.3 |
| 1.2 | yes | 0.5% | 119 | 6.2 |

The results in Table 1 show that HU-210 is capable of reducing histamine-induced itch when topically applied and is indicative of the capability of CBR activating agents to alleviate itch.

### Example 2

### Effect of Palmitoylethanolamide applied by patch on histamine-induced itch.

In this demonstration of whether a test material was a CBR activating agent, the method of Example 1 was followed except for employing palmitoylethanolamine (PEA, 100mM) in 50% ethylene glycol: 50% ethanol instead of HU-210 (in an aqueous Cream BP) at the specified concentration, and applied to the skin under a water-impermeable patch for 24 hours before the histamine challenge. The method was carried out four times. The results are summarised in Table 2 below.

**Table 2**

| Treatment | | Itch score (area under curve) | Itch score % of control |
|---|---|---|---|
| Control | Vehicle alone | 500 | 100 |
| 2.1 | Vehicle + PEA | 129 | 26 |
| Control | Vehicle alone | 377 | 100 |
| 2.2 | Vehicle + PEA | 103 | 27 |
| Control | Vehicle alone | 701 | 100 |
| 2.3 | Vehicle + PEA | 545 | 78 |
| Control | Vehicle alone | 56 | 100 |
| 2.4 | Vehicle + PEA | 32 | 57 |

From Table 2, it can be seen that the CBR activating ligand PEA can inhibit histamine-induced itch. The population exhibits a wide variation in response to histamine-induced itch. The same trend was observed for subjects who on average exhibited higher responses to the histamine-induced itch as for those who exhibited a much lower response.

### Example 3

### Effect of topically applied Palmitoylethanolamide on histamine-induced itch.

In this demonstration, Palmitoylethanolamide (50mM in ethanol) was mixed with Vaseline Intensive Care ^{™} Lotion (abbreviated to VICL)(volume ratio 1:1). This mixture was applied (250µl/ 5cm² - 5 times at 1h intervals) and histamine iontophoresis was performed on the treated site in the method described in Example 2. The vehicle control was ethanol:VICL (volume ratio 1:1). The procedure was carried out thrice, and the results summarised in Table 3 below.

**Table 3**

| Treatment | | Itch score (area under the curve) | Itch score % of control |
|---|---|---|---|
| Control | Vehicle alone | 3590 | 100 |
| 3.1 | Vehicle + PEA | 1186 | 33 |
| Control | Vehicle alone | 622 | 100 |
| 3.2 | Vehicle + PEA | 103 | 16.5 |
| Control | Vehicle alone | 264 | 100 |
| 3.3 | Vehicle + PEA | 211 | 79.7 |

The results in Table 3 show that PEA can inhibit histamine-induced itch when topically applied to the skin in the presence of a lotion. This response occurs irrespective of whether the subjects were high or low itch responders.

### Example 4 (Comparison)

### Effect of topically applied N-acyl ethanolamides on histamine-induced itch.

Related molecules stearylethanolamine (SEA; 50mM in ethanol) and linoleoylethanolamine (LAMEA; 50mM in ethanol) were tested as potential activating agents for a CBR, employing the test method described in Example 3 and applied in the ethanol:VICL lotion. The results are summarised in Table 4 below

**Table 4**

| Treatment | | Itch score Itch score % of (area under the control curve) | |
|---|---|---|---|
| Control | Vehicle alone | 1770 | 100 |
| 4.1 | Vehicle + SEA | 3354 | 190 |
| 4.2 | Vehicle + LAMEA | 1768 | 100 |
| Control | Vehicle alone | 210 | 100 |
| 4.3 | Vehicle + LAMEA | 356 | 169 |
| 4.4 | Vehicle + SEA | 485 | 231 |
| Control | Vehicle alone | 1383 | 100 |
| 4.5 | Vehicle + SEA | 3593 | 260 |
| 4.6 | Vehicle + LAMEA | 1497 | 108 |

From Table 4, it can be seen by comparison with the relevant control that other N-acylethanolamines SEA and LAMEA were unable to demonstrate any anti-pruritic action or even appeared to exacerbate the itch response.

Data from tests carried out on the scalps of healthy people and dandruff sufferers show that dandruff sufferers can experience greater sensitivity to histamine than those with healthy scalps. Therefore, this example and the preceding examples provide a good model for the effect of CB receptor activating agents on the scalp.

### Example 5

The following is an example of a shampoo composition according to the invention:

| **Ingredient** | **Example 5** |
|---|---|
| **Chemical Name** | **a.i. weight %** |
| SLES 2EO | 14 |
| Cocoamidopropylbetaine | 2 |
| Guar hydroxypropyltrimonium chloride | 0.1 |
| Dimethiconol | 1 |
| Crosslinked polyacrylic acid | 0.4 |
| Zinc pyrithione | 0.5 |
| Palmitoylethanolamide | 0.6 |
| Mica + titanium dioxide | 0.2 |
| Sodium benzoate | 0.5 |
| Water | to 100 |

## Claims

1. A hair and/or scalp treatment composition, comprising
- an antidandruff agent selected from zinc pyrithione, climbazole, ketoconazole, octopirox and mixtures thereof,
- from 0.5 to 10% by weight palmitoylethanolamide,
- and a cosmetically acceptable diluent or carrier.

2. A composition according to any preceding claim **characterised in that** it comprises from 0.01 to 30% by weight of the antidandruff agent.

3. A composition according to any preceding claim **characterised in that** it is a shampoo comprising a surfactant in an amount of from 3 to 50 wt%.

## Patentansprüche

1. Haar-und/oder Kopfhaut-Behandlungszusammensetzung, umfassend
- ein Antischuppenmittel, ausgewählt aus Zinkpyrithion, Climbazol, Ketoconazol, Octopirox und Gemischen davon,
- 0.5 bis 10 Gew.-% Palmitoylethanolamid
- und ein kosmetisch akzeptables Verdünnungsmittel oder einen kosmetisch akzeptablen Träger.

2. Zusammensetzung nach einem vorhergehenden Anspruch, **dadurch gekennzeichnet, daß** sie 0,01 bis 30 Gew.-% des Antischuppenmittels umfaßt.

3. Zusammensetzung nach einem vorhergehenden Anspruch, **dadurch gekennzeichnet, daß** sie ein Shampoo ist, umfassend ein oberflächenaktives Mittel in einer Menge von 3 bis 50 Gew.-%.

## Revendications

1. Composition de traitement des cheveux et/ou du cuir chevelu comprenant:
- un agent antipelliculaire choisi parmi le zinc pyrithione, le climbazole le kétoconazole, l'octopirox et leurs mélanges;
- de 0.5 à 10 % en poids de palmitoyléthanolamide;
- et un diluant ou un support cosmétiquement acceptable.

2. Compositon selon l'une quelconque des revendications précédentes, **caractérisée en ce qu'**elle comprend de 0,01 à 30% en poids de l'agent antipelliculaire.

3. Composition selon l'une quelconque des revendications précédentes, **caractérisée en ce qu'**elle est un shampoing comprenant un tensioactif dans une quantité allant de 3 à 50 % en poids.
